# EUROPÄISCHE PATENTSCHRIFT

(11) **EP 2 356 144 B1**
(45) Veröffentlichungstag und Bekanntmachung des Hinweises auf die Patenterteilung: **04.07.2018**
(21) Anmeldenummer: 09774854.5
(22) Anmeldetag: 17.11.2009
(51) Int. Cl.: C07K 16/18, C07K 14/47, A61P 25/28, C07K 7/08, A61K 39/00

(54) **ZUSAMMENSETZUNG ZUR HERSTELLUNG VON ANTIKÖRPERN GEGEN ANTI-AMYLOID BETA-PEPTID MIT D-PEPTIDEN**
COMPOSITION FOR PRODUCING ANTIBODIES BINDING TO ANTI-AMYLOID BETA PEPTIDE WITH D-PEPTIDES
COMPOSITION POUR LA PRÉPARATION D'ANTICORPS ANTI-PEPTIDE AMYLOÏDE BÊTA, COMPORTANT DES D-PEPTIDES

(30) Priorität: 19.11.2008 DE 102008037564
(43) Veröffentlichungstag der Anmeldung: 17.08.2011
(73) Patentinhaber: Forschungszentrum Jülich GmbH, 52425 Jülich (DE); Heinrich-Heine-Universität, 40225 Düsseldorf (DE)
(72) Erfinder: WILLBOLD, Dieter, 52428 Jülich (DE); KORTH, Carsten, 40219 Düsseldorf (DE); MÜLLER-SCHIFFMANN, Andreas, 51519 Odenthal (DE); FUNKE, Susanne Aileen, 52445 Titz (DE)
(74) Vertreter: Fitzner, Uwe
(86) Internationale Anmeldenummer: PCT/EP2009/065308
(87) Internationale Veröffentlichungsnummer: WO 2010/057882

(56) Entgegenhaltungen:
- WO-A1-00/52048
- WO-A1-94/05311
- WO-A2-02/081505
- WO-A2-02/096350
- NICOLAU C ET AL: "A LIPOSOME-BASED THERAPEUTIC VACCINE AGAINST BETA-AMYLOID PLAQUES ON THE PANCREAS OF TRANSGENIC NORBA MICE" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.022627199, Bd. 99, Nr. 4, 19. Februar 2002 (2002-02-19), Seiten 2332-2337, XP009062068 ISSN: 0027-8424
- SOLOMON B ET AL: "DISAGGREGATION OF ALZHEIMER BETA-AMYLOID BY SITE-DIRECTED MAB" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.94.8.4109, Bd. 94, 1. April 1997 (1997-04-01), Seiten 4109-4112, XP002942849 ISSN: 0027-8424
- WIESEHAN, KATJA: "Identification and characterization of a specific ligand for the Alzheimer amyloid-.beta.-peptide (A.beta.)" 2003, BERICHTE DES FORSCHUNGSZENTRUMS JUELICH , JUEL-4024, I-VII, 1-143 CODEN: FJBEE5; ISSN: 0944-2952 , XP002569790 Seite 70 Seite 119
- FINDEIS E A: "Modified peptide inhibitors of amyloid -beta-peptide polymerization" BIOCHEMISTRY, AMERICAN CHEMICAL SOCIETY, EASTON, PA.; US, Bd. 38, Nr. 21, 25. Mai 1999 (1999-05-25), Seiten 6791-6800, XP002143947 ISSN: 0006-2960
- TJERNBERG L O ET AL: "CONTROLLING AMYLOID BETA-PEPTIDE FIBRIL FORMATION WITH PROTEASE-STABLE LIGANDS" JOURNAL OF BIOLOGICAL CHEMISTRY, AMERICAN SOCIETY OF BIOLOCHEMICAL BIOLOGISTS, BIRMINGHAM, US, Bd. 272, Nr. 19, 9. Mai 1997 (1997-05-09), Seiten 12601-12605, XP002050230 ISSN: 0021-9258
- LEMERE CYNTHIA A ET AL: "Novel AP immunogens: Is shorter better?" CURRENT ALZHEIMER RESEARCH, Bd. 4, Nr. 4, September 2007 (2007-09), Seiten 427-436, XP8122216 ISSN: 1567-2050
- CAO C ET AL: "Mutant Amyloid-beta-sensitized dendritic cells as Alzheimer's disease vaccine" JOURNAL OF NEUROIMMUNOLOGY, ELSEVIER SCIENCE PUBLISHERS BV, XX LNKD- DOI:10.1016/J.JNEUROIM.2008.05.017, Bd. 200, Nr. 1-2, 30. August 2008 (2008-08-30), Seiten 1-10, XP024100005 ISSN: 0165-5728 [gefunden am 2008-07-22]
- REGENMORTEL VAN M H V ET AL: "D-PEPTIDES AS IMMUNOGENES AND DIAGNOSTIC REAGENTS", CURRENT OPINION IN BIOTECHNOLOGY, LONDON, GB, vol. 9, no. 4, 1 January 1998 (1998-01-01) , pages 377-382, XP000886984, ISSN: 0958-1669, DOI: 10.1016/S0958-1669(98)80011-6
- BIANCHI ELISABETTA ET AL: "A peptide vaccine based on retro-inverso beta-amyloid sequences fails to elicit a cross-reactive immune response.", ADVANCES IN EXPERIMENTAL MEDICINE AND BIOLOGY 2009, vol. 611, 2009, pages 363-364, ISSN: 0065-2598

## Beschreibung

Die Erfindung betrifft eine Zusammensetzung enthaltend D-Peptide oder Antikörper zur Verwendung als Therapeutikum bei der Behandlung von Krankheiten, bei denen eine aberrante Proteinaggregation oder -multimerisierung eine Rolle spielt. Ferner betrifft die Erfindung ein Verfahren zur Herstellung dieser Zusammensetzung sowie deren Verwendung.

Proteinaggregationserkrankungen bzw. die amyloide Degeneration ist eine heterogene Gruppe klinischer Zustände mit dem gemeinsamen Kriterium der in vielen Fällen aber nicht ausschließlich extrazellulären (systemisch oder lokalen) Ablagerung eines jeweils spezifischen Proteins in der geordneten Konformation von beta-Faltblattstruktur. Unter die Gruppe der Proteinaggregationserkrankungen bzw. Proteinfehlfaltungserkrankungen fällt auch die Alzheimer-Krankheit. Die Alzheimer-Krankheit (lateinisch Morbus Alzheimer) tritt als neurodegenerative Erkrankung in ihrer häufigsten Form bei Personen über dem 65. Lebensjahr auf. Die Pathogenese ist geprägt von einer Verschlechterung der kognitiven Leistungsfähigkeit, die meistens begleitet wird von einer Abnahme der täglichen Aktivitäten, mit Verhaltensauffälligkeiten und neuropsychologischen Symptomen. Die Patienten verlernen im fortgeschrittenen Stadium altbekannte Fertigkeiten und erkennen nahe stehende Personen nicht mehr wieder. Die Lebenserwartung nach einer Alzheimer-Diagnose beträgt aus statistischer Sicht im Schnitt etwa sieben bis zehn Jahre.
Ferri *et al.* zeigen, dass im Jahr 2005 ca. 24 Millionen Menschen von Demenz betroffen sind, wovon ca. 60% auf Morbus Alzheimer zurückzuführen ist (Ferri et al., Lancet. 366, Nr. 9503, 2005, S. 2112-7). Der Morbus Alzheimer ist derzeit die häufigste Demenzerkrankung für die bis zum heutigen Stand (Oktober 2008) keine kausale Therapie besteht.

Ein pathologisches Kennzeichen von Morbus Alzheimer, welches auch schon vor ersten klinischen Symptomen bestimmbar ist, sind Plaques (sog. Alzheimer-Fibrillen). Diese Proteinaggregate bestehen größtenteils aus fehlerhaft gefaltetem Amyloid beta-Peptid (auch Abeta-Peptid oder Amyloid beta-Peptid) und lagern sich im Gehirn von Alzheimer-Patienten ab (Walsh und Selkoe; 2007, Journal of Neurochemistry 101:1172-1184) und sind die Folge einer erhöhten Anhäufung des Amyloid beta-Peptids im Gehirn. Allerdings stellen die Amyloid beta-Peptidfibrillen nur das Endstadium eines Prozesses dar, der mit der Abspaltung von monomerem Amyloid beta-Peptid aus APP (amyloid precursor protein) beginnt, dann neurotoxische Amyloid beta-Peptid Oligomere ausbildet, und erst dann mit Amyloid beta-Peptidfibrillen endet.

Um die Anhäufung mit dem Amyloid beta-Peptid zu verhindern, gibt es als experimentelle Therapien die aktive und passive Immunisierung mit dem Amyloid beta-Peptid-Fragment als Immunogen.

Experimentelle Therapien werden in *in vitro* Modellen der Amyloid beta-Peptid Aggregation, in Zellmodellen der Produktion des Amyloid beta-Peptids, sowie in transgenen Mausmodellen die im Gehirn Amyloid beta-Peptidaggregate bilden, durchgeführt. Insbesondere wurden aktive und passive Immunisierung als Therapie in dem transgenen Mausmodell der Amyloid beta-Peptidaggregation durchgeführt (Schenk et al., Nature, 1999; Bard et al. 2003) In der Erstbeschreibung der aktiven Immunisierung wurde volle Länge L-Amyloid beta-Peptid als Immunogen verwendet und führte in einem Mausmodell der Alzheimererkrankung im Rahmen einer Immunisierung zu einer Clearance der Plaques (Schenk et al., Nature, 1999). In einem ähnlichen Mausmodell wurden auch die passive Immunisierung erfolgreich angewendet. Dabei zeigte sich, dass N-terminale Epitope im Bereich Amyloid beta-Peptid 1-11 besonders effizient zur cerebralen Clearance der Amyloid beta-Peptidfibrillen führten (Bard et al.; 2003, PNAS 100 (4):2023-2028). Derzeit finden zwei Studien mit passiver Immunisierung, d.h. Antikörpern / Antikörperfragmenten bei Patienten mit Morbus Alzheimer statt (Brody und Holtzman; 2008, Annual Reviews in Neuroscience 31:175-193).

Die Immunisierung wurde in Tierversuchen erfolgreich getestet , jedoch führte die aktive Immunisierung beim Menschen zu einer T-Zell-vermittelten Autoimmunantwort bzw. Autoimmunkrankheit, bei der das körpereigene Immunsystem im Gehirn des Patienten Meningoenzephalitiden (Meningoenzephalitis) herbeiführte (Brody und Holtzman; 2008, Annual Reviews in Neuroscience 31:175-193). Daher wird die aktive Immunisierung mit dem Amyloid beta-Peptid-Fragment derzeit als wenig erfolgversprechend angesehen.

Antikörper gegen das Amyloid beta-Peptid für die passive Immunotherapie wurden von Bard et al (PNAS 100(4):2023-2028), Dodel et al. (2002; Ann Neurol 52:253-256) und 2007 von Gardberg et al. (PNAS 104(40):15659-15664) gegen die Aminosäuren 1-8 des Amyloid beta-Peptids beschrieben. Eine Immunantwort in Mäusen hervorgerufen durch eine palmitoylierte A-beta-(1-16)-Sequenz von Nicolai et al (PNAS 99(4), 2002. 2332-2337) beschrieben.

Die Immunisierung von alten Beagle-Hunden mit vollständigen (1-42) fibrillaren Amyloid beta-Peptid wurde beispielsweise auch von Head et al. (2008; The Journal of Neuroscience 28(14):3555-3566) beschrieben. Jedoch gibt es schwerwiegende Unterschiede zwischen Hunden und Menschen in Bezug auf die Verwendung des Amyloid beta-Peptids als Immunogen, da im Gegensatz zum Menschen in Hunden keine entgegenwirkende Autoimmunantwort auftritt.

Cribbs et al. (1997; The Journal of Biological Chemistry 272(11):7431-7436) untersuchten den Mechanismus des Amyloid beta-Peptides und verwendeten dabei die D- und L-Isomere des vollständigen Amyloid beta-Peptides sowie einer verkürzten Form (Aminosäuren 1-42 bzw. 25-35). Die Stereospezifität wird ebenfalls von Esler *et al.* untersucht. Den Nachweis von Proteinen mit veränderter Konformation / Prionen durch die Verwendung von Sequenzen, die dem nachzuweisenden Target-Protein entsprechen zeigen Orser et al. (US 2008/0171341 A1).
Willbold et al. (WO 02/081505 A2) zeigen die Durchführung eines Phagendisplays zur Identifizierung von Aminosäuresequenzen die an das Ass-Peptid binden. Findeis et al. (US 6,689,752 B2) untersuchen den Einfluss von Sequenzen bestehend aus 3-5 Aminosäuren auf die Aggregation des Amyloid beta-Peptides.

Cribbs *et al.* folgerten aus ihren Ergebnissen, dass die Neurotoxizität nicht stereoisomer-spezifisch ist. Jedoch wird nicht auf eine Immunantwort hin getestet, aber auf die geringere Immunogenizität der D-Isomere verwiesen.

Geylis *et al.* beschreiben Zelllinien, gewonnen von gesunden Menschen, welche Antikörper synthetisieren die an die Aminosäuren 1-16 des Amyloid beta-Peptides binden und diskutieren die Verwendung bei der passiven Immunisierung. Von Lee et al. (2005; American Neurological Association 58:430-435) wurden die Antikörper-Seren von Menschen verglichen, die nach einer Amyloid beta-Peptid-Immunisierung an Meningoenzephalitis erkrankten und nicht erkrankten. Ein Ergebnis dieser Untersuchung war, dass die Antikörper hauptsächlich gegen die Aminosäuren 1-8 des Amyloid beta-Peptides gerichtet waren.

**Aufgabe der vorliegenden Erfindung** ist daher die Bereitstellung einer Zusammensetzung zur Verwendung als Therapeutikum bei der vorbeugenden Behandlung bzw. Therapie von Proteinaggregations/fehlfaltungserkrankungen. Die Zusammensetzung sollte in einem Verfahren zur aktiven Immunisierung gegen Morbus Alzheimer und anderen Proteinaggreagtionserkrankungen eingesetzt werden können. Dabei soll die selektive Evozierung einer B-Zell Immunantwort unter Vermeidung einer T-Zellimmunantwort erreicht werden.

**Diese Aufgabe wird erfindungsgemäß gelöst** durch eine Zusammensetzung enthaltend D-Peptide mit SEQ ID NO: 2 oder Antikörper die an diese Sequenz und an das Amyloid-Peptid binden.

Die Zusammensetzung, enthält somit ein D-Peptid als Immunogen, welches mit einem Amyloid beta-Peptid interagiert und die Antikörperbildung von Anti-Amyloid beta-Peptid-Antikörpern hervorruft. Die Zusammensetzung kann auch einen Amyloid beta-Peptid-interagierenden Antikörper enthalten, wobei dieser Antikörper in der Lage ist, an das zuvor genannte D-Peptid zu binden und zusätzlich an das Amyloid beta-Peptid. Bei der "Interaktion" zwischen dem D-Peptid und dem Amyloid beta-Peptid handelt es sich um eine Protein-Proteinwechselwirkung.

Die erfindungsgemäße "Zusammensetzung" kann z. B. ein Impfstoff, ein Medikament (z. B. in Tablettenform), eine Injektionslösung, ein Nahrungs- oder Nahrungsergänzungsmittel sein. Die erfindungsgemäße Zusammensetzung kann lediglich aus D-Peptiden oder lediglich aus Antikörpern bestehen, verbunden mit den zu der jeweiligen Applikation benötigten Hilfsmitteln wie z. B. Salze wie AluminiumSalze, Puffer oder Lösungsmittel.

Weitere Vorteile, die sich gegenüber dem Stand der Technik ergeben, sind:
- ein geringes Molekulargewicht des Immunogens
- keine T-Zellantwort (zelluläre Autoimmunität) und damit deutlich weniger Nebenwirkungen
- die Verwendung von geringen Mengen der Zusammensetzung ist ausreichend, da das Immunogen verzögert abgebaut wird
- das Immunogen ist einfach zu modifizieren oder zu kombinieren.

Durch die erfindungsgemäße Zusammensetzung kommt es nach der Immunisierung mit D-Peptiden zur Generierung von Amyloid beta-Peptid-spezifischen Antikörpern. Diese Immunisierung mit Amyloid beta-Peptid-fremden Immunogen aus D-Peptid ist dem Amyloid beta-Peptid Immunogen überlegen, da eine Antikörperantwort gegen das Amyloid beta-Peptid evoziert wird, ohne die gleichzeitige, Nebenwirkungsbelastende anti-Amyloid beta-Peptid T-Zellimmunantwort.

Unter "Immunisierung" versteht man das Erzeugen einer Immunantwort gegen ein definiertes Antigen mit dem Ziel dieses Antigen zu eliminieren, zu neutralisier und/oder anderweitig für den Organismus ungefährlich zu machen. Aktive Immunisierung: Einbringen eines Antigens (meist einer Peptidsequenz) dergestalt, dass eine endogen Immunantwort erzeugt wird, die zu der Eliminierung des Antigens, und v.a. verwandter Antigene führt.

Unter "Passive Immunisierung" versteht man das Einbringen von Antikörpern parenteral, die zu einer Eliminierung des Antigens führen.

Mit der erfindungsgemäßen Zusammensetzung kann die Bildung von Amyloid beta-Peptid-Multimeren durch Bindung eines Antikörpers an die Multimerisierungsdomäne des Amyloid beta-Peptids verhindert werden, wobei der Antikörper nach Immunisierung mit einem D-Peptid gebildet wird.

"Amyloid beta-Peptid-Multimere" bedeutet im Sinne der Erfindung die stabile Zusammenlagerung mehrerer Amyloid beta-Peptidmolekülen mit der Erlangung neuer Funktionen ("gain fo function").

Der Begriff "Multimerisierungsdomäne" definiert diejenigen Domänen des Amyloid beta-Peptids, die mit der Interaktion der Amyloid beta-Peptide untereinander zu tun haben. In einer Variante erfüllen die Aminosäuren 10-42 des Amyloid beta-Peptids diese Funktion.

Weiterer Indikationsbereich ist die Verwendung bei der Therapie von Morbus Alzheimer, Diabetes mellitus und andere Amyloiderkrankungen, sowie Erkrankungen, bei denen die Homo-Multimerisierung von einem Protein entscheidend ist, wie z. B. Morbus Parkinson, frontotemporale Demenz, amyotrophe Lateralsklerose, Mukoviszidose, bestimmte Formen der Epilepsie.

"D-Peptide" bestehen in einer Variante aus einer retro-inversen Sequenz zum Amyloid beta-Peptid oder Amyloid beta-Peptid-Teilfragmenten und vollständig aus D-Aminosäuren.

Ein "Teilfragment" besteht aus 3, 4, 5, 6, 7, 8, 9, 10, 11, 12, 13, 14, 15, 16 oder mehr Aminosäuren homolog zur Aminosäuresequenz des Amyloid beta-Peptids.

In einer weiteren Variante binden die erfindungsgemäßen D-Peptide an die Multimerisierungsdomäne des Amyloid beta-Peptids. In einer weiteren Variante weisen die D-Peptide die Sequenz SEQ ID NO:2 auf und bestehen vollständig aus D-Aminosäuren. In einer weiteren Variante weisen die D-Peptide die Sequenz SEQ ID NO:2 auf, und enthalten teilweise L-Aminosäuren. In einer weiteren Variante weisen die D-Peptide homologe Sequenzen zu SEQ ID NO:2 auf. Unter "D-Peptid" wird ein Peptid verstanden, welches sich aus Aminosäuren in der D-Form zusammensetzt.

Enthalten "teilweise" L-Aminosäuren bedeutet, dass 1, 2, 3, 4, 5, 6, 7, 8, 9, 10 oder mehr Aminosäuren homolog zur Aminosäuresequenz des aus D-Aminosäuren bestehenden D-Peptids durch jeweils die gleiche Aminosäure in der L-Konformation ersetzt sind.

"Homologe Sequenzen" bedeutet im Sinne der Erfindung, dass eine Aminosäuresequenz eine Identität mit der SEQ ID NO:2 von mindestens 70%, 75%, 80%, besonders bevorzugt 85%, 90%, insbesondere 91%, 92%, 93%, 94%, 95%, 96%, 97%, 98% oder 99% aufweist. Homologe Sequenzen zu den erfindungsgemäßen Sequenzen bestehend aus D-Aminosäuren können auch teilweise L-Aminosäuren enthalten. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) beechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidsequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Diese homologen D-Peptide binden entweder an das Amyloid beta-Peptid und/oder die Amyloid beta-Peptid-Multimerisierungsdomäne. Sie können aus dem Amyloid beta-Peptid abgeleitet sein (homolog) und sind funktional definiert, d.h. fähig eine mindestens dem Amyloid beta-Peptid identische Immunantwort zu evozieren. Sie sind aber nicht das Amyloid beta-Peptid selber und erzeugen eine Antikörperantwort in Mäusen gegen das Amyloid beta-Peptid und/oder Amyloid beta-Peptid-Multimeren.

Unter dem Begriff "Retro-inverses Peptid" oder "Retro-inverso Peptid" wird erfindungsgemäß ein Peptid bezeichnet, welches sich aus Aminosäuren in der D-Form zusammensetzt (invers: Chiralität des alpha-C-Atoms invers zur L-Form), bei dem zusätzlich die Sequenzreihenfolge zum ursprünglichen Peptid umgekehrt wurde (retro = rückwärts; siehe auch Regenmortel und Muller, 1998; Current Opinion in Biotechnology 9:377-382).

Weiterer Gegenstand der Erfindung ist eine Zusammensetzung enthaltend Antikörper, wobei der Antikörper an das Amyloid Peptid oder Amyloid beta-Peptid bindet und
a) an eine retro-inverse Sequenz des Amyloid beta-Peptids oder Amyloid beta-Peptid-Teilfragmenten bindet und/oder
b) an die Multimerisierungsdomäne des Amyloid beta-Peptids bindet und auch an das Amyloid beta-Peptid und/oder
c) an SEQ ID NO:2 oder homologe Sequenzen davon mit einer Identität von mindestens 70% zu SEQ ID NO:2 bindet und an das Amyloid beta-Peptid.

Vorteilhafterweise weisen die Antikörper Eigenschaften des Amyloid beta-Peptids selber auf und kompetitieren mit der Multimerisierung des Amyloid beta-Peptids, wenn zur Antikörper-Generierung Peptide, die an die Amyloid beta-Peptid-Multimerisierungsdomäne binden, verwendet werden.

Weiterer Gegenstand der Erfindung ist ein Verfahren zur Herstellung der erfindungsgemäßen Zusammensetzung, wobei durch Immunisierung von Tieren bzw. Tierzellen und Fusionierung von Tierzellen mit Myelomzellen und darauffolgender Selektion und Kultivierung antikörperproduzierende Hybridzellen erhalten werden und Antikörper isoliert und aufgereinigt werden.

Die Immunisierung, Fusionierung, Selektion, die Kultivierung von antikörperproduzierenden Hybridzellen und die Antikörper-Isolierung bzw. Antikörper-Aufreinigung kann zum Beispiel mit den in in "Monoclonal antibodies" (Heddy Zola. Springer Verlag, New York 2000) gezeigten und dem Fachmann bekannten Methoden durchgeführt werden. Weitere Verfahren, insbesondere für das Screening von antikörperproduzierenden Zellen, wurden von Korth et al., 1999, in Methods in Enzymology 309:106f gezeigt.

Zur Immunisierung können Tiere (bzw. Tierzellen), z. B. Mäuse, Kaninchen, Ratten, Meerschweinchen und weitere, standardmäßig zur Antikörperherstellung eingesetzte Tiere, verwendet werden. Werden Mäuse für die Immunisierung mit der erfindungsgemäßen Zusammensetzung gewählt, kann eine Immunisierungsstrategie wie folgt aussehen: die Zusammensetzung kann dabei subkutan injiziert werden (z. B. dreimal; Tag 0: komplettes Freund-Adjuvans, Tag 21 und 22: inkomplettes Freund-Adjuvans; Blutabnahme ca. 100 µl am Tag 31). Nach einer oder mehreren BoosterInjektionen mit der erfindungsgemäßen Zusammensetzung werden die Mäuse beispielsweise am Tag 50 betäubt, enthauptet und es wird die Milz entnommen. Die daraus gewonnenen Milzzellen (Splenocyten) werden mit Maus-Myelom-Zellen im Verhältnis 1:5 vermischt und durch Hinzugabe von 50% Polyethylen-Glycol (PEG) (8 Minuten, Raumtemperatur) fusioniert. Danach werden die Zellen gewaschen und über-Nacht kultiviert. Die Selektion auf die Antikörper-produzierende HybridomaZellen kann z. B. in HAT-Medium und in 96-well Mikrotiter-Platten erfolgen und im ELISA-Enzymtest.

Weiterer Gegenstand der Erfindung ist eine Verwendung einer erfindungsgemäßen Zusammensetzung zur Verhinderung von Amyloid beta-Peptid-Multimeren. Dabei können D-Peptide oder Antikörper, die an die Multimerisierungsdomäne des Amyloid beta-Peptids binden, verwendet werden

In einer erfindungsgemäßen Variante enthält die Zusammensetzung 10-1000 µg Immunogen. In einer weiteren Variante enthält die Zusammensetzung 20-900, 25-750, 30-600, 40-500, 50-400, 50-300 oder 50-250 µg Immunogen. Denkbar wäre auch eine Zusammensetzung die weniger als 10 µg Immunogen oder mehr als 1000 µg Immunogen enthält.

Vorzugsweise wird unter "Immunogen" ein zur Immunität führendes Antigen verstanden. Ein Immunogen ist ein Stoff, der in der Lage ist, eine Immunantwort auszulösen. Immunogene unterscheiden sich dadurch von den Antigenen, die von einem Antikörper erkannt werden, von denen aber nicht alle allein eine Immunantwort auszulösen können.

Vorzugsweise wird unter "Immunität" die durch Immunisierung herbeigeführte und durch Auftreten spezifischer Antikörper und/oder Zellen gekennzeichnete veränderte Reaktionsbereitschaft des Immunsystems gegenüber Antigenen (wie z. B. Viren, Bakterien oder Fremdeiweiß) verstanden.

Weiterer Gegenstand der Erfindung ist eine Verwendung der erfindungsgemäßen Zusammensetzung bei der Vorbeugung und/oder Therapie von Morbus Alzheimer. So können D-Peptide, die an die Multimerisierungsdomäne des Amyloid beta-Peptids binden, zur Herstellung eines Medikamentes zur Vorbeugung und/oder Therapie von Morbus Alzheimer dienen.

Weiterer Gegenstand der Erfindung ist eine Verwendung der erfindungsgemäßen Zusammensetzung enthaltend D-Peptide als Therapeutikum und/oder zur Krankheitsvorbeugung von Morbus Alzheimer.

Weiterer Gegenstand der Erfindung ist eine Verwendung einer erfindungsgemäßen Zusammensetzung enthaltend Antikörper als Therapeutikum und/oder zur Krankheitvorbeugung oder zur Diagnose von Morbus Alzheimer.

Weiterer Gegenstand der Erfindung ist eine Verwendung einer Zusammensetzung enthaltend Antikörper zur Diagnose von Morbus Alzheimer, zum Nachweis oder Quantifizierung von Biomolekülen oder zur Lokalisierung von Biomolekülen in Geweben, Zellen oder bei der Erkennung spezieller Zelltypen enthält.

Unter Nachweis bzw. Quantifizierung von Biomolekülen im Sinne der Erfindung wird hierbei die Bestimmung von Substanzen durch Antigen-Antikörper-Reaktionen verstanden (z. B. Immunoassays).

Unter der Lokalisierung von Biomolekülen in Geweben bzw. Zellen oder unter der Erkennung spezieller Zelltypen im Sinne der Erfindung wird hierbei die Nutzung von Antikörpern z. B. in der Immunhistochemie oder Immuncytochemie verstanden.

Vorteil der vorliegenden Erfindung ist, dass eine therapeutische Antikörperantwort evoziert wird, ohne dass das dabei eingesetzte D-Peptid von T-Zellen verarbeitet wird.

Somit wurde eine Methode erfunden, mit deren Hilfe auf einfache Weise Immunisierungsstrategien gegen Proteinaggregationserkrankungen bzw. Proteinkonformationserkrankungen entwickelt werden können. So kann in einem Verfahren im Zusammenhang mit der Therapie von Morbus Alzheimer, bei dem mit Amyloid beta-Peptid-interagierenden D-Peptiden immunisiert wird, in einem Individuum eine Amyloid beta-Peptid-Toxizität neutralisierende und/oder Amyloid beta-Peptid clearende humorale Immunantwort erreicht werden. Ferner können Verfahren entwickelt werden, bei dem es sich um eine andere Krankheit als Morbus Alzheimer handelt, bei der die Aggregation bzw. Multimerisierung von Proteinen ebenfalls krankheitsrelevant ist und bei dem an Multimerisierungs-Interfaces bindende D-Peptide verwendet werden (Proteinkonformationserkrankungen des ZNS, Diabetes usw.).

Vorzugsweise wird unter "Clearing", "Clearance" bzw. "clearend" die Entfernung einer bestimmten exogenen oder endogenen Substanz aus einem Gewebe als spezifische Leistung eines Organs (z.B. renale Clearance), einer zellulären Komponente (z.B. Makrophagen, Mikroglia), oder subzellulären Kompartments (z. B. Proteasom) verstanden.

Ein weiterer Vorteil der Erfindung ist, dass zusätzlich der Epitopraum ("epitope space") des Amyloid beta-Peptids verlassen wird, der grundsätzlich bei hoher Selbsttoleranz die humorale Immunantwort limitieren kann. Bei der erfindungsgemäßen Verwendung von Amyloid beta-Peptid-bindenden D-Peptiden wird zudem die T-Zellimmunantwort unterdrückt, da diese Peptide nicht degradiert und präsentiert werden können.

Offenbart werden auch Antikörper, die sich gegen die Amyloid beta-Peptid-Multimerisierungsdomäne richten und die Multimerisierung des Amyloid beta-Peptids verhindern. Danach können, durch die Markierung mit dem Antikörper, Amyloid beta-Peptide bzw. die zusammengelagerten Amyloid beta-Peptid-Ablagerungen durch das Körper-eigene Immunsystem abgebaut werden. Weitere erfindungsgemäße Antikörper richten sich gegen das Amyloid beta-Peptid allgemein und nicht gegen die Multimerisierungsdomäne im speziellen.

### Beispiele

Im Folgenden wird die Erfindung anhand der Ausführungsbeispiele und der beiliegenden Abbildungen näher beschrieben.

Abbildung 1 zeigt einen Western Blot von synthetischem Amyloid-beta-Peptid (1) und CHO-Zellen, mit APP transfiziert (3); irrelevante Kontrolle (2). Dieser Western Blot zeigt, dass 9A6 kein APP oder Amyloid-beta-Peptid auf dem Western Blot erkennt (linke Seite), während der universale anti-Amyloid-beta-Peptid-Antikörper IC16 dies vermag (rechte Seite).

Abbildung 2 zeigt einen Western Blot einer Immunpräzipitation von IC16 (rechte Seite) und 9A6 (rechte Seite) vom Überstand permanent APP transfizierter CHO-Zellen, die das Amyloid-beta-Peptid sezernieren. Es ist zu sehen, dass 9A6 Amyloid-beta-Peptid Oligomere erkennt.

Abbildung 3 zeigt eine immunohistochemische Färbung mit 9A6 einer kortikalen Probe eines Patienten mit Morbus Alzheimer. Es ist zu sehen, dass der 9A6 Antikörper schwach Amyloid-beta-Peptid Plaques erkennt.

### Beispiel 1 (Immunisierung von Mäusen mit D3 Peptid)

Mäuse wurden mit D-Peptiden immunisiert. Es wurden das D3-Peptid verwendet, sowie ein retro-inverses Peptid des Amyloid beta-Peptids (riAbeta1-16; retro-inverso-Abeta(1-16)). Nach mehreren Boosterinjektionen (Auffrischungsimpfungen, Booster-Effekt) wurde eine Fusion zur Erzeugung monoklonaler Antikörper nach Standardmethoden durchgeführt. Es konnten monoklonale Antikörper isoliert werden, die an D3, riAbeta1-16 und an das Amyloid beta-Peptid binden. Somit führt die Immunisierung mit den genannten D-Peptiden zu einer protektiven Immunantwort gegen das Amyloid beta-Peptid.

Vorzugsweise wird unter "Booster-Effekt" (erzielt durch eine Boosterinjektion bzw. Auffrischungsimpfung) eine immunologische Sekundärreaktion bzw. eine verstärkte Immunantwort nach einem Wiederholungskontakt mit dem Antigen verstanden.

Die zum Immunisieren verwendeten Peptide hatten folgende Sequenzen:
"D3"-Peptid: rprtr Ihthr nr (SEQ ID NO:1)
retro-inverso-Abeta(1-16): kqhhv eygsd hrfea d (SEQ ID NO:2)

D3 wurde an KLH (Keyhole Limpet Hämocyanin) kovalent verbunden (crosslinking) und mit Standardprozeduren Mäusen immunisiert. Diese wurden dann nach Standardprotokollen mit Mausmyelomzellen fusioniert. Der Zellkulturüberstand der entstandenen Hybridomazellen wurde dann sowohl auf die Erkennung des D3 Peptids und des Amyloid beta-Peptids im Standard ELISA getestet.

Folgende Klone erkennen ausschließlich D3 (und nicht das Amyloid beta-Peptid): 4G11, 13H11, 32A11, 40B7

Folgende Klone erkennen sowohl D3 als auch das Amyloid beta-Peptid: 9A6, 14B5, 39B12

Diese Experimente zeigen, dass
1. die Immunisierung mit D3 eine Antikörperantwort gegen das Amyloid beta-Peptid zufolge hat und
2. dass es an bestimmten Antikörpern eine Bindungsstelle gibt, die sowohl das Amyloid beta-Peptid als auch D3 erkennen kann.

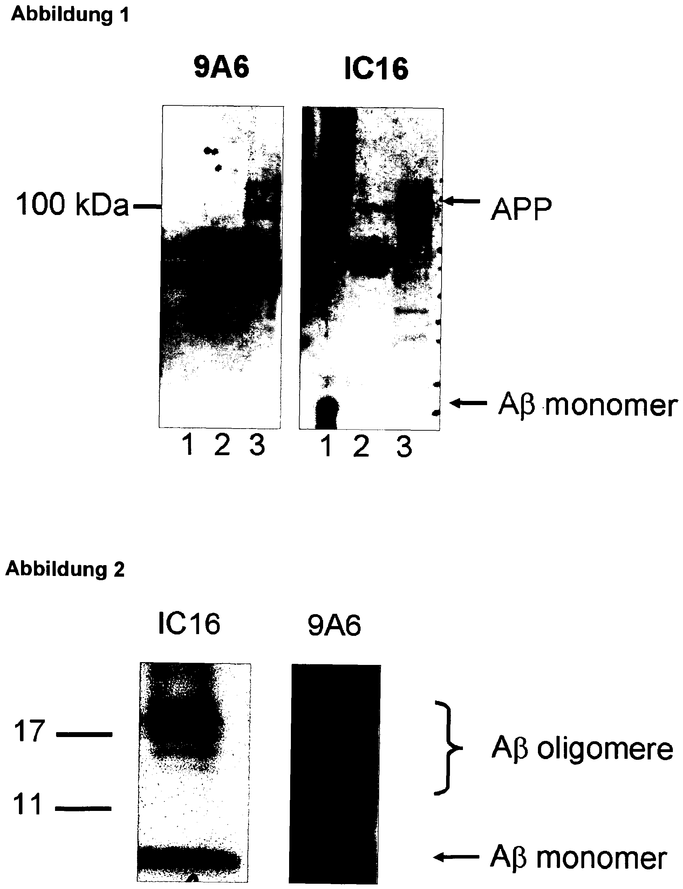

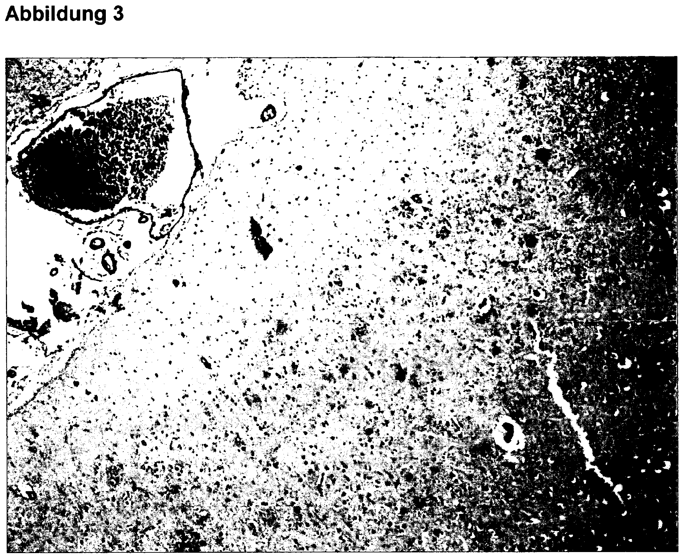

Der exemplarisch untersuchte antiD3/anti-Amyloid beta-Peptid Antikörper 9A6 wurde weiter in den in Abbildung 1, Abbildung 2 und Abbildung 3 gezeigten Assays getestet.

### SEQUENCE LISTING

<120> Zusammensetzung zur Herstellung von anti-Amyloid beta-Peptid-Antikörpern mit D-Peptiden
   <130> FZJ0801DE
   <160> 2
   <170> PatentIn version 3.4
<210> 1
   <211> 12
   <212> PRT
   <213> Artificial
   <220>
   <223> D3-Peptid (willbold)
   <400> 1 
arg pro arg thr arg leu his thr his arg asn arg
1               5                   10
<210> 2
   <211> 16
   <212> PRT
   <213> Artificial
   <220>
   <223> retro-inverso-Aβ(1-16); riAβ(1-16)
   <400> 2

lys gln his his val glu tyr gly ser asp his arg phe glu ala asp
1               5                   10                  15

## Patentansprüche

1. Zusammensetzung enthaltend D-Peptide mit SEQ ID NO: 2 oder Antikörper die an diese Sequenz und an das Amyloid-Peptid binden zur Verwendung als Therapeutikum und/oder zur Krankheitsvorbeugung.

2. Zusammensetzung zur Verwendung gemäß Anspruch 1, **dadurch gekennzeichnet, dass** der Antikörper
a) an eine retro-inverse Sequenz des Amyloid beta-Peptids oder Amyloid beta-Peptid-Teilfragmenten bindet und an das Amyloid beta-Peptid und/oder
b) an die Multimerisierungsdomäne des Amyloid beta-Peptids bindet und/oder
c) an SEQ ID NO:2 oder homologe Sequenzen davon mit einer Identität von mindestens 70% zu SEQ ID NO:2 bindet und an das Amyloid beta-Peptid.

3. Verfahren zur Herstellung der Zusammensetzung zur Verwendung gemäß Anspruch 1, **dadurch gekennzeichnet, dass** durch Immunisierung von nicht menschlichen Tieren bzw. Tierzellen und Fusionierung von Tierzellen mit Myelomzellen und darauffolgender Selektion und Kultivierung antikörperproduzierende Hybridzellen erhalten werden und Antikörper isoliert und aufgereinigt werden.

4. Zusammensetzung zur Verwendung gemäß Anspruch 1 zur Verhinderung von Amyloid beta-Peptid-Multimeren zur Behandlung von Morbus Alzheimer.

5. Zusammensetzung zur Verwendung gemäß Anspruch 1 bei der Vorbeugung und/oder Therapie von Morbus Alzheimer.

6. Zusammensetzung zur Verwendung gemäß Anspruch 1 enthaltend D-Peptide als Therapeutikum und/oder zur Krankheitsvorbeugung von Morbus Alzheimer.

7. Zusammensetzung zur Verwendung gemäß Anspruch 1 enthaltend Antikörper als Therapeutikum und/oder zur Krankheitsvorbeugung oder zur Diagnose von Morbus Alzheimer.

8. Zusammensetzung zur Verwendung gemäß Anspruch 1 enthaltend Antikörper zur Diagnose von Morbus Alzheimer, zum Nachweis oder Quantifizierung von Biomolekülen oder zur Lokalisierung von Biomolekülen in Geweben, Zellen oder bei der Erkennung spezieller Zelltypen.

## Claims

1. A composition comprising D-peptides having the sequence SEQ ID NO:2 or antibodies which bind to this sequence and to the amyloid peptide for use as a therapeutic and/or for disease prevention.

2. The composition for the use as claimed in claim 1, **characterised in that** the antibody
a) binds to a retro-inverse sequence of the amyloid beta peptide or amyloid beta peptide subfragments and to the amyloid beta peptide and/or
b) binds to the multimerization domain of the amyloid beta peptide and/or
c) binds to SEQ ID NO:2 or homologous sequences thereof with an identity of at least 70% to SEQ ID NO:2 and to the amyloid beta peptide.

3. A process for preparing the composition for use as claimed in claim 1, **characterised in that** antibody-producing hybrid cells are obtained by immunizing non human animals or animal cells and fusing animal cells with myeloma cells with subsequent selection and culturing, and antibodies are isolated and cleaned up.

4. Composition for use as claimed in claim 1 for preventing amyloid beta peptide multimers for the treatment of Alzheimer's disease.

5. Composition for use as claimed in claim 1 in the prevention and/or therapy of Alzheimer's disease.

6. Composition for use as claimed in claim 1 , comprising D-peptides as a therapeutic and/or for the disease prevention of Alzheimer's disease.

7. Composition for the use as claimed in claim 1 , comprising antibodies as a therapeutic and/or for the disease prevention or diagnosis of Alzheimer's disease.

8. Composition for the use as claimed in claim 1 comprising antibodies for the diagnosis of Alzheimer's disease, for the detection or quantification of biomolecules or for the localization of the biomolecules in tissues, cells or in the recognition of specific cell types.

## Revendications

1. Composition comportant des D-peptides avec la séquence SEQ ID NO:2 ou des anticorps se liant à cette séquence et au peptide amyloïde destiner à l'utilisation en tant que produit thérapeutique et/ou pour prévenir des maladies.

2. Composition destinée à l'utilisation selon la revendication 1, **caractérisée en ce que** l'anticorps
a) se lie à la séquence rétro-inverse du peptide amyloïde bêta ou des peptides amyloïde bêta sous- fragments et au peptide amyloïde bêta et/ou
b) se lie à la domaine de multimérisation du peptide amyloïde bêta et/ou
c) se lie à la SEQ ID NO:2 ou aux séquences homologues de çelle-çi avec une identité d'au moins 70% de la SEQ ID NO:2 et au peptide amyloïde bêta.

3. Un procédé pour la préparation de la composition destiner à l'utilisation selon la revendication 1, **caractérisée en ce que** des cellules hybrides que produisent des anticorps, sont obtenues par l'immunisation des animaux non humains ou des cellules des animaux et par fusion des cellules des animaux avec des cellules du myélome avec selection subséquente et par mise en culture, et des anticorps sont isolés et purifiés.

4. Composition destinée à l'utilisation selon la revendication 1 pour prévénir des multimères de peptides amyloïde bêta pour le traitement de la maladie d' Alzheimer.

5. Composition destinée à l'utilisation selon la revendication1 en tant que produit de prévention et/ou thérapeutique de la maladie d'Alzheimer.

6. Composition destinée à l'utilisation selon la revendication1 comprenant des D-peptides en tant que produit thérapeutique et/ou de prévention de la maladie d' Alzheimer.

7. Composition destinée à l'utilisation selon la revendication1 comprenant des anticorps en tant que produit thérapeutique et/ou de prévention de maladie ou pour le diagnose de la maladie d'Alzheimer.

8. Composition destinée à l'utilisation selon la revendication1 comprenant des anticorps pour le diagnose de la maladie d'Alzheimer, pour la détection ou quantification des biomolécules ou pour la localisation des biomolécules dans les tissus, des cellules ou dans la détection des types de cellules spéçifiques.
